**Europäisches Patentamt**

**European Patent Office**

**Office européen des brevets**

(19)

(11) Veröffentlichungsnummer: **0 087 756**
**A2**

(12) # EUROPÄISCHE PATENTANMELDUNG

(21) Anmeldenummer: **83101783.5**

(22) Anmeldetag: **23.02.83**

(51) Int. Cl.³: **A 61 N 1/36**

(30) Priorität: **26.02.82 DE 3207006**

(43) Veröffentlichungstag der Anmeldung:
**07.09.83 Patentblatt 83/36**

(84) Benannte Vertragsstaaten:
**DE FR GB NL**

(71) Anmelder: **SIEMENS AKTIENGESELLSCHAFT**
**Berlin und München Wittelsbacherplatz 2**
**D-8000 München 2(DE)**

(72) Erfinder: **Elmqvist, Hakan, Dr.**
**Sunnedalsvägen 7**
**S-161 38 Bromma(SE)**

(54) **AV-sequenzieller Herzschrittmacher.**

(57) Bei einem AV-sequenziellen Herzschrittmacher ist ein steuerbares Verzögerungsglied vorgesehen für die Einstellung der Zeit zwischen Vor- und Herzkammeraktivität (AV-Zeit). Um mit einem derartigen Herzschrittmacher Tachykardien möglichst rasch beenden zu können, ist ein Detektor zum Erfassen derartiger Tachykardien vorgesehen, über den das Verzögerungsglied derart angesteuert ist, dass im Falle einer Tachykardie die AV-Zeit geändert wird. Die Änderung ist dabei so gewählt, dass die neue AV-Zeit zur Beendigung der Tachykardie geeignet ist. Nach einer vorbestimmten Zeit kehrt das Verzögerungsglied wieder auf die ursprüngliche AV-Zeit zurück.

EP 0 087 756 A2

Croydon Printing Company Ltd.

SIEMENS AKTIENGESELLSCHAFT
Berlin und München

Unser Zeichen
VPA 82 P 7302 E

**0087756**

## AV-sequenzieller Herzschrittmacher

Die Erfindung betrifft einen AV-sequenziellen Herzschrittmacher mit einem steuerbaren Verzögerungsglied für die Einstellung der Zeit zwischen Vor-und Herzkammeraktivität (AV-Zeit). Unter AV-sequenziellen Herzschrittmachern sollen dabei alle Typen von Herzschrittmachern verstanden werden, die in der Herzkammer stimulieren und durch ein Vorkammersignal gesteuert sind oder die in der Herzkammer und der Vorkammer stimulieren und durch ein Vorkammersignal gesteuert sind oder auch solche, die sowohl in der Vor- als auch in der Herzkammer stimulieren und auch durch beide Kammersignale gesteuert werden. Ein derartiger Herzschrittmacher ist beispielsweise in der US-PS 4 108 148 beschrieben. Mit Hilfe des Verzögerungsgliedes kann dabei die hämodynamisch effektive PQ-Zeit der Herzfrequenz angepasst werden.

Der vorliegenden Erfindung liegt die Aufgabe zugrunde, einen Herzschrittmacher der eingangs genannten Art derart zu erweitern und zu verbessern, dass behandelbare Tachykardien, beispielsweise Reentry-Tachykardien, verhindert oder zumindest schnell beendet werden können.

Diese Aufgabe wird erfindungsgemäss dadurch gelöst, dass ein Detektor zum Erfassen einer vorliegenden Tachykardie oder bereits der Ankündigung einer solchen vorgesehen ist, dass der Detektor in diesem Fall ein Ausgangssignal erzeugt, das auf das Verzögerungsglied gegeben ist und eine Änderung der AV-Zeit bewirkt, wobei die Änderung so gewählt ist, dass die neue AV-Zeit zur Verhinderung oder Beendigung der Tachykardie geeignet ist, und dass das Verzögerungsglied nach einer vorbestimmten Zeit wieder auf die ursprüngliche AV-Zeit zurückkehrt.

Es sind bereits nicht AV-sequenzielle Herzschrittmacher bekannt, mit denen eine Tachykardie beendet werden kann (britische Patentanmeldung 7907998 oder US-PS 3942534). Zum Erkennen einer Tachykardie ist dabei ein spezieller Detektor vorgesehen. Beim Auftreten einer solchen Tachykardie werden zusätzliche Stimulierungsimpulse an das Herz abgegeben. Um eine Tachykardie zu beenden, muss man das Herz zu einem Zeitpunkt stimulieren und damit in eine refraktäre Phase überführen, so dass die zirkulierende Aktivierung, die die Tachykardie verursacht, unwirksam wird. Aus der US-PS 3942534 ist dazu bekannt, die zeitlichen Abstände zwischen den Stimulierungsimpulsen kontinuierlich nach jedem Herzzyklus zu verändern, entweder zu verkürzen oder zu verlängern. Bei einer genügend grossen Zahl von Stimulierungsimpulsen sollte dann einer den richtigen Zeitabstand zu den Herzaktivitäten haben, um die Tachykardie zu beenden. Aus der genannten britischen Anmeldung ist es bekannt, die auf einen derartigen Stimulierungsimpuls folgende Herzaktivität zur gezielten Zeitsteuerung für den nächsten Stimulierungsimpuls zu verwenden.

Es sind auch AV-sequenzielle Herzschrittmacher zur Behandlung von Tachykardien bekannt. Diese haben jedoch stets eine feste Verzögerungszeit, unabhängig, ob eine Tachykardie vorliegt oder nicht. Das führt dazu, dass die Patienten ständig unter hämodynamischen Beschwerden leiden.

Mit dem Herzschrittmacher gemäss der vorliegenden Erfindung lassen sich nunmehr Tachykardien auf besonders vorteilhaft einfache Weise beenden, indem die Stimulierung in der Herzkammer mit einer bestimmten Zeitverzögerung zu dem Ereignis in der Vorkammer vorgenommen wird. Umgekehrt ist es ohne Einschränkung auch möglich, die Steuersignale aus der Herzkammer zu entnehmen und die Stimulierung in der Vorkammer vorzunehmen. Wesentlich für die Erfindung ist dabei das Zustandekommen einer veränderbaren Verzögerungszeit zwischen den Herzaktivitäten in einer Kammer und den Stimulierungsimpulsen in der anderen Kammer. Erreicht wird das gemäss der vorliegenden Erfindung durch einen Detektor zum Erfassen von Tachykardien, dessen Ausgangssignal zum Steuern der Verzögerungszeit auf das Verzögerungsglied gegeben wird. Im einfachsten Fall kann die Verzögerungszeit beim Auftreten einer Tachykardie auf einen einzigen fest vorgegebenen Wert übergehen.

Um die Anwendungsmöglichkeit eines derartigen Herzschrittmachers zu erweitern, ist vorteilhaft vorgesehen, dass der Detektoreingang wahlweise mit der Vor- oder der Herzkammerelektrode verbindbar ist. Weiterhin kann das Verzögerungsglied derart aufgebaut sein, dass es nach der durch eine auftretende Tachykardie erfolgten Ansteuerung mit der dadurch verursachten veränderten Verzögerungszeit nach einer fest vorgegebenen Zeit wieder auf die ursprüngliche Verzögerungszeit zurückkehrt. Sollte die Tachykardie immer noch vorliegen, steuert der Detektor das Verzögerungsglied erneut an.

**0087756**

In einer vorteilhaften Weiterbildung der Erfindung ist vorgesehen, dass die Verzögerungszeit nach Beendigung der Tachykardie auf den ursprünglichen Wert zurückkehrt. Hierbei wird das veränderte Ausgangssignal des Detektors nach Beendigung einer Tachykardie zum erneuten Ansteuern des Verzögerungsgliedes benutzt.

Wie bereits ausgeführt, kann die beim Vorliegen einer Tachykardie gewählte Verzögerungszeit fest vorgegeben sein. Ebenso ist es jedoch möglich, diese Verzögerungszeit nach einem vorgebbaren Muster zu verändern, beispielsweise nach jedem Herzzyklus um einen vorbestimmten Betrag zu verkleinern oder zu vergrössern. Eine besonders schnelle Beendigung einer Tachykardie erreicht man gemäss der vorliegenden Erfindung dadurch, dass die Veränderung der AV-Zeit eine Funktion der elektrophysiologischen Reaktion des Herzens auf frühere Stimulierungsimpulse ist.

Sowohl fest vorgegebene Verzögerungszeiten oder nach einem bestimmten Muster veränderbare können entweder durch den Aufbau des Verzögerungsgliedes festgelegt sein oder aber von ausserhalb des Herzschrittmachers auf drahtlosem Wege einstellbar, insbesondere programmierbar sein.

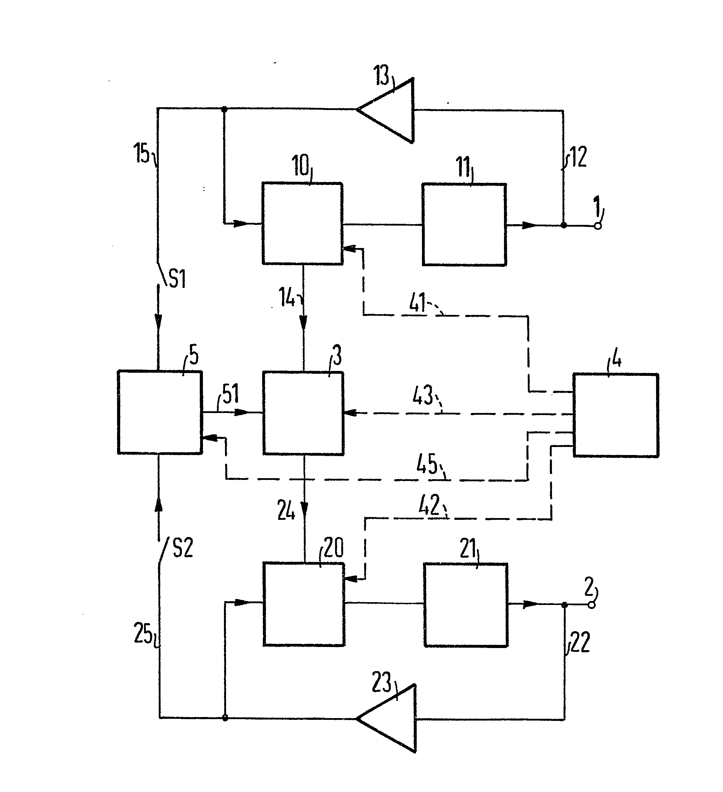

Anhand einer Figur wird im folgenden ein Ausführungsbeispiel der Erfindung näher beschrieben und erläutert. Die Figur zeigt dabei schematisch in einem Blockschaltbild die wesentlichen Elemente des erfindungsgemässen Herzschrittmachers.

In der Figur sind zwei Anschlüsse 1 bzw. 2 dargestellt, die jeweils mit einer Elektrode verbunden werden können. Dabei soll die an den Anschluss 1 angeschlossene Elektrode der Vorkammer (Atrium) Impulse zuführen können und die an den Anschluss 2 angeschlossene Elektrode der Herzkammer (Ventrikel). Die Impulse für beide Anschlüsse werden in bekannter Weise von Impulsgeneratoren 10 bzw. 20 erzeugt und über Ausgangsschaltstufen 11 bzw. 21 freigegeben. Die Stimulierungselektroden dienen gleichzeitig zum Abfühlen der Herztätigkeit.

Die derart empfangenen Signale werden über Leitungen 12 bzw. 22 und Verstärker 13 bzw. 23 den Impulsgeneratoren 10 bzw. 20 als Steuersignale zugeführt. Weiterhin enthält der Herzschrittmacher ein Verzögerungsglied 3, das zur Steuerung der Verzögerungszeit zwischen der Herzaktivität in einer Kammer und einem Stimulierungsimpuls in der anderen Kammer dient. Im vorliegenden Ausführungsbeispiel wird das Verzögerungsglied 3 über eine Leitung 14 vom Impulsgenerator 10 angesteuert, wie durch den Pfeil in der Leitung 14 angedeutet ist.

Diese Ansteuerung kann beispielsweise erfolgen, wenn der Impulsgenerator 10 einen Stimulierungsimpuls an die Vorkammer abgibt oder wenn durch die entsprechende Elektrode eine spontane Aktivität in der Vorkammer angezeigt wird. Nach einer bestimmten, einstellbaren Verzögerungszeit gibt das Verzögerungsglied 3 über eine Leitung 24 ein Ausgangssignal an den Impulsgenerator 20 ab. Erst nach Erhalt eines derartigen Signales wird ein vom Impulsgenerator 20 erzeugter Stimulierungsimpuls für die Weiterleitung an die Anschlussklemme 2 und damit die Herzkammerelektrode freigegeben. Auch der umgekehrte Fall ist denkbar, indem der Impulsgenerator 20 ein Steuersignal für das Verzögerungsglied abgibt, das wiederum die Auslösung der Impulse des Impulsgenerators 10 freigibt. Die Pfeile in den Leitungen
14 bzw. 24 sind dabei umzudrehen.

Wie durch strichlinierte Pfeile 41 bis 43 angedeutet
ist, können in den Impulsgeneratoren 10 bzw. 20 und
in dem Verzögerungsglied 3 Funktionsparameter beispielsweise über eine externe Programmiereinheit 4 verändert werden. Eine derartige Programmiereinheit ist
beispielsweise aus dem Siemens-Elema Prospekt ME 372/
5406, 1979, bekannt. Wie eine derartige Programmierung
vorgenommen wird, ist beispielsweise aus der DE-AS
22 36 434 oder der DE-OS 29 39 197 bekannt.

Über Leitungen 15 bzw. 25 sowie Schalter S1 bzw. S2
können die über die Elektroden erfassten und durch die
Verstärker 13 bzw. 23 verstärkten Herzsignale einem
Tachykardiedetektor 5 zugeführt werden. Durch die Schalter S1 bzw. S2 kann der Detektor wahlweise die Vorkammer- oder die Herzkammersignale analysieren. Ein derartiger Tachykardiedetektor ist beispielsweise aus der
US-PS 3942534 bekannt. Das Ausgangssignal des Tachykardiedetektors 5 wird über eine Leitung 51 als zusätzliches Steuersignal auf das Verzögerungsglied 3 gegeben. Wie über einen weiteren strichlinierten Pfeil 45
angedeutet ist, können auch die Funktionsparameter des
Tachykardiedetektors, beispielsweise seine Empfindlichkeit, von ausserhalb des Herzschrittmachers über die
Programmiereinheit verändert werden.

Die Funktionsweise des Herzschrittmachers gemäss dem
vorliegenden Ausführungsbeispiel ist folgende:

Solange vom Tachykardiedetektor 5 keine Tachykardie

0087756

angezeigt wird, arbeitet die Schaltung wie bei bekannten AV-sequenziellen Herzschrittmachern. Solange von
ausserhalb des Herzschrittmachers keine Änderungen der
Parameter vorgenommen werden, liegt damit die Verzögerungszeit fest. Unter Umständen kann jedoch die Verzögerungszeit bereits davon abhängen, ob die Herzaktivitäten in der Vorkammer spontan oder durch einen Stimulierungsimpuls angeregt sind.

Wird nun eine Tachykardie registriert oder ein ektopischer (an falscher Stelle des Herzens angeregter)
Herzschlag, der einer beginnenden Tachykardie vorausgeht, so erzeugt der Tachykardiedetektor 5 ein Ausgangssignal, das zu einer Änderung der Verzögerungszeit (AV-
Zeit) des Verzögerungsgliedes führt. Die in einem solchen Fall gewählte Verzögerungszeit kann in bekannter
Weise eine einzige fest vorgegebene verkürzte Zeit oder
eine nach einem Muster veränderbare Verzögerungszeit
sein. Ist die Tachykardie beendet, springt das Ausgangssignal des Detektors 5 wieder auf seinen ursprünglichen
Wert zurück. Gleichzeitig kehrt auch die Verzögerungszeit wieder auf den Ausgangswert zurück.

Der besondere Vorteil dieser Schaltung besteht darin,
dass bereits die Vorankündigung einer Tachykardie abgefühlt werden kann und unmittelbar Gegenmassnahmen
ergriffen werden können, so dass die Tachykardie überhaupt erst gar nicht auftritt. Wird also beispielsweise
durch den Tachykardiedetektor 5 ein derartiger ektopischer Herzschlag detektiert, der ohne Gegenmassnahmen
zu einer zirkulierenden Aktivierung Vorkammer-Herzkammer-Vorkammer führen würde, d.h. also zu einer Tachykardie, so kann mit einer gezielten kurzen Verzögerungszeit zu dieser ektopischen Herzaktivität in der Vorkammer ein Stimulierungsimpuls an die Herzkammer abgegeben werden, der diese in eine refraktäre Phase versetzt und damit für das aus der Vorkammer ankommende Aktivivierungssignal unempfänglich macht. Die weitere Aktivierung wird dadurch blockiert.

Weiterhin besteht ein Vorteil darin, dass durch die Erfassung der Aktivitäten in einer Kammer und die Steuerung der Verzögerungszeit in Abhängigkeit von dieser Aktivität die zur Beendigung einer Tachykardie vorgesehenen Stimulierungsimpulse in ihrem Zeitverhalten besser festgelegt werden können, wodurch sich die Chancen für eine schnelle und wirksame Beendigung einer Tachykardie weiter erhöhen.

1 Figur
7 Patentansprüche

Patentansprüche

1. Av-sequenzieller Herzschrittmacher mit einem steuerbaren Verzögerungsglied für die Einstellung der Zeit zwischen Vor- und Herzkammeraktivität (AV-Zeit), d a d u r c h g e k e n n z e i c h n e t , dass ein Detektor (5) zum Erfassen einer vorliegenden Tachykardie oder bereits der Ankündigung einer solchen vorgesehen ist, dass der Detektor (5) in diesem Fall ein Ausgangssignal erzeugt, das auf das Verzögerungsglied (3) gegeben ist und eine Änderung der AV-Zeit bewirkt, wobei die Änderung so gewählt ist, dass die neue AV-Zeit zur Verhinderung oder Beendigung der Tachykardie geeignet ist, und dass das Verzögerungsglied (3) nach einer vorbestimmten Zeit wieder auf die ursprüngliche AV-Zeit zurückkehrt.

2. Herzschrittmacher nach Anspruch 1, d a d u r c h g e k e n n z e i c h n e t , dass der Detektoreingang wahlweise mit der Vor- oder Herzkammerelektrode (1,2) verbindbar ist.

3. Herzschrittmacher nach einem der Ansprüche 1 oder 2, d a d u r c h g e k e n n z e i c h n e t , dass die Verzögerungszeit nach Beendigung der Tachykardie auf den ursprünglichen Wert zurückkehrt.

4. Herzschrittmacher nach einem der Ansprüche 1 bis 3, d a d u r c h g e k e n n z e i c h n e t , dass die AV-Zeit nach einem vorgebbaren Muster variierbar ist.

5. Herzschrittmacher nach einem der Ansprüche 1 bis 4, d a d u r c h g e k e n n z e i c h n e t , dass die

Art der AV-Zeitveränderung von ausserhalb des Herzschrittmachers einstellbar, insbesondere programmierbar
ist.

6. Herzschrittmacher nach einem der Ansprüche 4 oder 5,
d a d u r c h   g e k e n n z e i c h n e t , dass die
AV-Zeit in bekannter Weise stufenweise nach jedem Herzzyklus um einen vorbestimmten, insbesondere programmierbaren Betrag verändert wird.

7. Herzschrittmacher nach einem der Ansprüche 4 bis 6,
d a d u r c h   g e k e n n z e i c h n e t , dass die
Veränderung der AV-Zeit in bekannter Weise eine Funktion der elektrophysiologischen Reaktion des Herzens
auf frühere Stimulierungsimpulse ist.